# EUROPEAN PATENT APPLICATION

(11) **EP 3 120 874 A1**
(43) Date of publication of application: **25.01.2017**
(21) Application number: 15715326.3
(22) Date of filing: 19.03.2015
(51) Int. Cl.: A61L 9/20

(54) **SYSTEM FOR THE TREATMENT OF FATS**

(30) Priority: 21.03.2014 ES 201430399
(71) Applicant: Wismok Esp Barcelona S.L., 08019 Barcelona (ES)
(72) Inventor: PEREZ TOMAS, Amador, E-08019 Barcelona (ES); COBO DELGADO, Jacob, E-08019 Barcelona (ES); GALLEN MELENDO, Joaquim, E-08019 Barcelona (ES)
(74) Representative: Isern-Jara, Nuria
(86) International application number: PCT/ES2015/070196
(87) International publication number: WO 2015/140382

(57) **Abstract**

A system for the treatment of fats, such as those that can be carried preferably in fumes or fumes products, in a kitchen or food industry installation, and which can be linked to the extraction and/or conduction installation of said fumes, comprising at least a source (4) of UV radiation, which can be UVA and preferably of a wavelength of 315 to 380 nm, preferably of a laser type, pulsed light or LED (for example a LED of gallium nitride). Sources are preferably placed on the wall or ceiling of the flue, so that they are accessible from the outside.

## Description

The present invention refers to a system for the treatment of fats, preferably such as those that may be carried in fumes or fumes products occurring in kitchens and the food industry, which uses UV radiation as a means of disinfection and degreasing.

It may be used in the manufacture of industrial, domestic and/or commercial fumes extractors, as well as in the food industry.

### Known state of the art

In the state of the art, the use of ultraviolet (UV) light as a surface disinfectant is known, normally by using a mercury lamp that emits at a wavelength in the 254 nm range.

However, some ranges of UV radiation involve a series of unavoidable risks. Moreover, the mercury in the lamps is a very toxic heavy metal, for which reason the system generates dangerous wastes.

Moreover, the mercury lamps are very large and hence require that the treatment system be defined on the basis of these lamps, which must be centred in the flow, which generates deposits of fats and other highly-inflammable substances.

### Brief disclosure of the invention

The invention consists of a system for treating fats, such as those that can be carried preferably in fumes or fumes products, as defined in the claims.

It should be noted that in the present application the term "fat" is to be understood as a synonym of triglycerides.

Specifically, the invention comprises at least one UV radiation source, particularly LEDs, pulsed light or laser, which preferably emit UVA radiation at a wavelength that preferably will be between 315 and 380 nm, much less dangerous than UVC or UVB radiation, both for the eyes and for the skin. In addition, UVA radiation does not generate ozone, which increases user safety. This source is linked to an installation to extract and/or conduct the fats generated in kitchen facilities.

The purifying and sterilising effect of UV radiation, as well as the demonstrated effect of removing fats, permits installation in other equipment, such as air conditioners located in kitchen and other environments and that therefore may be susceptible to absorbing and conducting fumes that may carry fats.

UVA radiation, apart from purifying, sterilising and eliminating germs, generates OH radicals that adhere to the fat chains and has been shown to decompose them. In this way, not only are the walls cleaned, but adhesion of the fat chains to the walls of the flue is avoided. Using the present system, we succeed in accelerating a totally natural process. The greater the irradiation, the greater the rate of decomposition. The use of UVA radiation, using wavelengths between 315 and 380 nm, succeeds in an unexpected and effective way in breaking the molecular chain of fatty components present, for example, in fumes from culinary or industrial environments.

The wavelength of the invention also offers very good results in the elimination of odours due to fats contained in the treated fumes. It is effective for all types of fats originating in cooking, including chicken fat, which, because of its composition, is very hard to dissolve.

In a specially preferred form, the fats to be decomposed are of the triglyceride type.

Thanks to the above characteristics, a device is produced for the treatment of fats that may be carried preferably in fumes, which device does not require adding catalysers or replacing canisters or cartridges containing reactants or other products in order to break the molecular chain of the fats.

Moreover, ozone is not generated during or after the reaction. Another advantage of the present invention is that it is easy to adapt and install in installations for the conduction and treatment of fat-carrying fumes and gases.

In distinction to the state of the art, relatively large-sized devices are not required and the object of the invention does not have to be centred in the fumes flow.

The radiation sources are located preferably in the wall or ceiling of the flue of the installation used to extract or conduct said fumes, so that they do not offer resistance to the passage of the fumes or form fat deposits that are difficult to clean and dangerous because of their flammability. In addition, since they are not in the direct fumes flow, they will have to withstand a lower temperature, which is favourable for LEDs, which normally work at less than 100°C, although diamond-based prototypes exist that can withstand up to 300°C. Preferably LEDs of gallium nitride (GaN) are used in the invention.

They are placed before or after the filter in the fumes flue, or even on both sides.

They may be placed in only one wall or in the ceiling or in both walls, facing each other in series but alternating so that they will cover the greatest possible surface area without overlapping. For this purpose, lenses can be installed that modify the radiation beam that is emitted. It is convenient to place them in pass-through holes so that they are accessible from the outside of the flue, while maintaining the air-tightness of the flue through the location of the radiation sources (including brackets).

For safety reasons, the device can include an emitter of coloured light (violet, for example) in order to warn that it is in operation.

In air-conditioning installations, the LED UVA sources are placed in the inside walls of this equipment in such a way that, when pulsated by specially-designed electronic programs, they can cover the surfaces to be treated, benefiting from the effects already described as the object of this invention with regard to the removal of fats and odours in a special manner, as well as because of their germicidal and purification effects.

### Description of the drawings.

For a better understanding of the invention, the following figures are included:

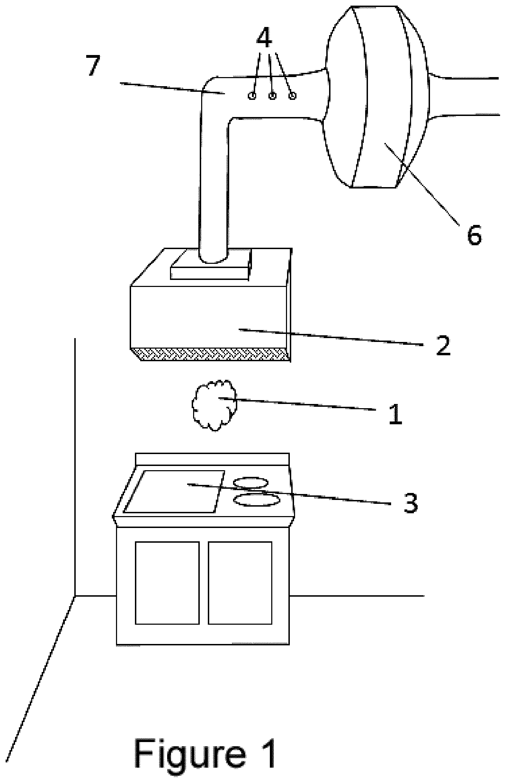

Figure 1 is a schematic drawing of a kitchen with the system for the treatment of fumes and products thereof resulting from the invention.
Figures 2A and 2B are schematic drawings of the placement of the UV radiation sources in the flue.
Figures 3A and 3B are images of the results obtained with the invention in a first test.
Figures 4A and 4C are results of gas chromatography in a second test of the invention.
Figure 5 shows the results of gas chromatography for triglycerides in the second test of the invention.

### Embodiments of the invention.

An embodiment of the invention will be described briefly below, as an illustrative but not limiting example thereof.

The invention consists of a system for the treatment of the fats (1) that may be carried preferably in fumes or the resulting products thereof, which may be used in the installation to extract and/or conduct the fumes situated after the extractor (2) of a kitchen (3) or industrial food-processing plant. Generally, it is located before the extraction pump in the flue (7).

The system targets fats carried by the fumes, while simultaneously helping to clean conduits by radiating walls and floor in order to break down the adhered fats. In addition, it prevents adhesion of new fat deposits to the flue (7).

The system is based on the use of UV-light sources (4), preferably UVA with wavelengths of 315-380 nm, located in the wall of the flue (7). Preferably, the sources (4) will be accessible from outside the flue (7), for example, by making a pass-through hole in which the sources (4) are placed, closing the perimeter so as make it air-tight.

The sources (4) will be laser, pulsed light or LEDs, particularly gallium nitride LEDs.

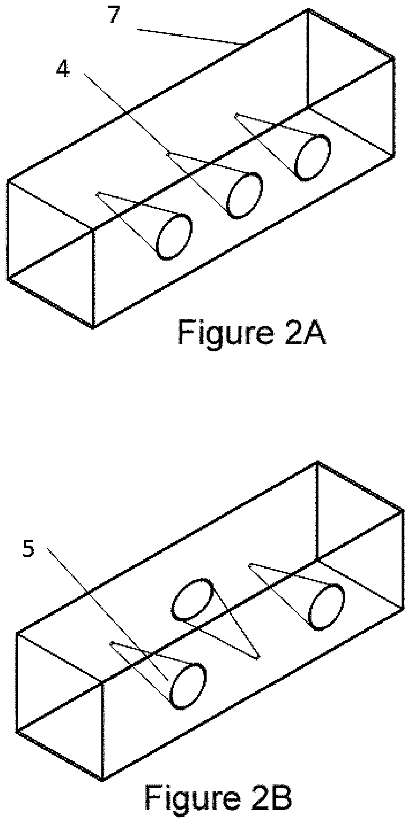

The sources (4) may be placed in a single wall or in the ceiling of the flue (figure 2A) or in both walls alternately (figure 2B), preferably in such a way their beams do not overlap but are controlled optimally throughout the section of flue and wall.

In order to control the beams (5) better, the system may place lenses (not shown) in front of the sources (4). Normally, the lenses will be 60° lenses.

Preferably, the system also comprises a filter (6) that may be located before, between or after the sources (4), the advantages of each solution being obvious to an expert in the field.

It is recommended that safety measures, such as a coloured light emitter or a pilot light in the extractor hood, be installed to permit knowing when the latter is in operation and to assure that the UVA radiation cannot escape the flue by placing an impermeable material (such as plastic) as a protective screen.

The use of the indicated wavelength has proven unexpectedly to be particularly beneficial for the elimination of odours through decomposition of the fats, as shown in the tests that are provided.

### Test 1

Samples of fats (A, B, C) coming from the extractor hood of a chicken rotisserie are placed in three Petri dishes. A control sample is placed in another Petri dish.

The control sample is kept in a dark place at room temperature, while the other samples are placed in a reproduction of a fumes flue at 20°C with a source (4) of 365-nm UVA radiation located 50 cm away. The source (4) focuses directly on sample (B), while the other samples receive less radiation. It is calculated that it receives an irradiance of 0.018 mW/cm² and a UVA radiation dose of 22.28 J/cm² at the end of 14 days.

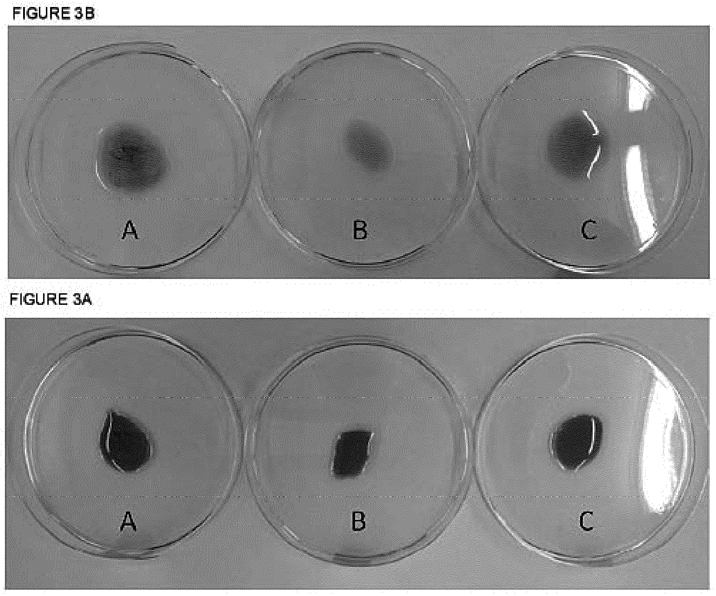

After 45 and 116 hours, significant decolouration and less viscosity are observed and the odour is much less than in the control sample. Figures 3A and 3B show the Petri dishes initially and after 116 hours and a reduction in size and colour may be seen, particularly in sample B.

### Test 2

Samples of fats coming from the extractor hood of a chicken rotisserie are placed in three Petri dishes. One of the three is kept as a control sample and the others are irradiated with an LED source (4) of 365 nm UVA radiation with a strength such that they receive an irradiance of 0.12 mW/cm². The samples are irradiated for 72 and 133 hours respectively. The samples receive, respectively, UVA radiation doses of 29.84 J/cm² and 54.72 J/cm².

The samples are analysed using gas chromatography and qualitative and semi-quantitative (intercomparison) analysis of the fats and glycerides being performed.

In order to analyse the fatty acids, 50 mg of the sample are placed and saponified with 10 ml of KOH 6% in methanol. It is left to react overnight at room temperature. It is acidified with hydrochloric acid and extracted with 15 ml of n-hexane (x 3). The extract is evaporated in a rotary vaporiser until almost dry. Fifteen ml of boron trifluoride/methanol are added and it is left to react overnight in darkness at room temperature. Fifteen ml of Milli-Q ultra-pure water (Millipore Corporation) are added and it is extracted with n-hexane (x3). It is concentrated to 1 ml and injected in the chromatograph. The carrier gas is helium at 1 ml/min. Analysis of the triglycerides is performed by placing 50 mg of the sample in a 1.8 ml vial. To this are added 200 µl of MSTFA (n-methyl n-trimethylsilyl trifluoroacetamide) and 200 µl of pyridine. They are left at room temperature until the time when they are injected into a chromatograph. The carrier gas is still helium, but at 1.3 ml/min.

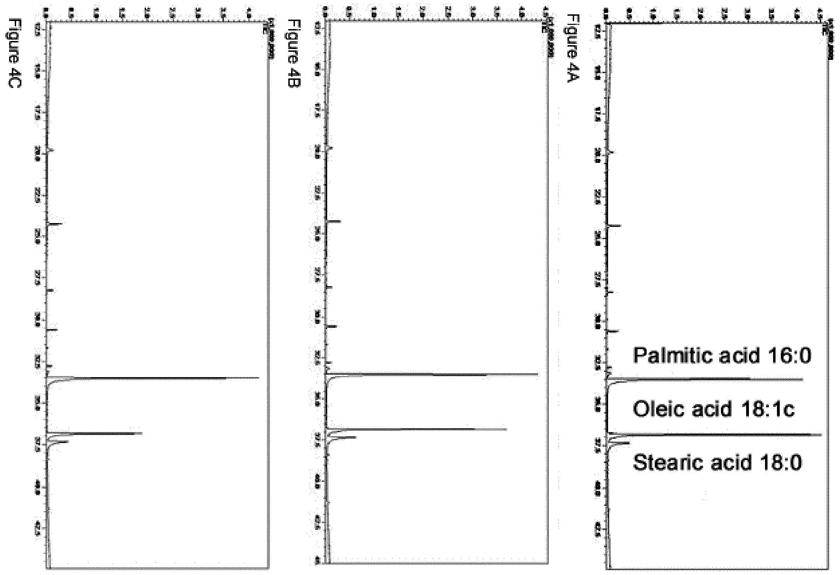

Both the fatty acid profiles obtained after saponification and the glyceride profiles show a gradual reduction in the peak areas of the treated samples with respect to the control sample. Figures 4A (control), 4B (72 hours) and 4C (133 hours) show the changes with regard to fatty acids.

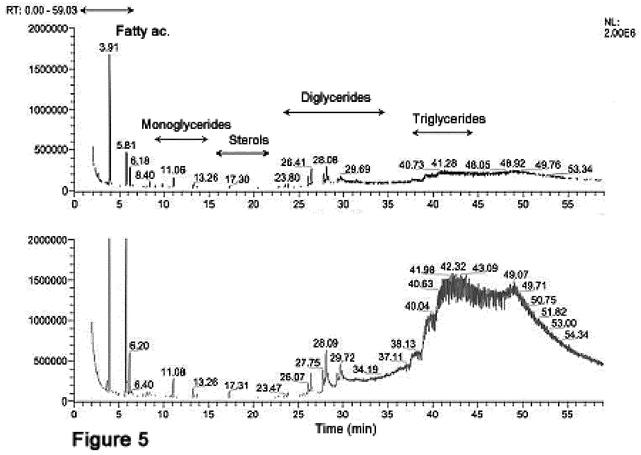

Figure 5 in turn shows the result for triglycerides for the original sample (below) and after 133 hours (above).

In addition, it was found that there was a reduction in the weight of the samples treated for 72 hours and 133 hours with the UVA radiation dose specified above.

The present invention may be installed in fumes flues that carry fats, extractor hoods, air conditioning installations that may absorb such fat-carrying fumes (not shown) etc. and may be present in numbers from one UVA radiation source (4) to a number or them, the number being adapted to the particular needs of each case.

It is not considered necessary to make this description more extensive in order for an expert in the field to understand its scope and operation, as well as the advantages that are derived in comparison to the state of the art.

## Claims

1. A system for the treatment of fats (1), preferably such as those that may be carried in fumes or fumes products usually generated in kitchen facilities, **characterised by** the fact that it comprises at least a source (4) of UV radiation, which can be linked to an extraction and/or conduit installation of said fumes, wherein the radiation is UVA radiation.

2. A system of claim 1, **characterised in that** the wavelength of radiation is comprised between 315 and 380 nm.

3. A system according to any of the above claims, **characterised in that** the source (4) is a LED light.

4. A system according to claim 3, **characterised in that** the LED light is a LED of gallium nitride or of an alloy thereof.

5. A system according to any of claims 1-2, **characterised in that** the source (4) is of a laser type.

6. A system according to any of claims 1-2, **characterised in that** the source (4) is pulsed light.

7. A system, according to any of the above claims, **characterised in that** the extraction and/or conduction installation comprises at least one flue.

8. A system, according to claim 7, **characterised in that** is has a filter (6) in the flue (7).

9. A system, according to claim 7 or 8, **characterised in that** at least one source (4) is placed on the wall or ceiling of the flue (7).

10. A system, according to claim 9, **characterised in that** it has more than one source (4) en a single wall or ceiling.

11. A system, according to claim 9, **characterised in that** it has more than one source (4) on the two walls, in an alternate manner.

12. A system, according to any of claims 9 to 11, **characterised in that** the sources (4) are placed in pass-through holes in the flue (7), while maintaining the air-tightness of said sources (4).

13. A system, according to any of the above claims, **characterised in that** it comprises a source of coloured light.

14. A system, according to any of the above claims, **characterised in that** it comprises a lens in at least one source (4).
